# EUROPEAN PATENT SPECIFICATION

(11) **EP 2 508 199 B1**
(45) Date of publication and mention of the grant of the patent: **17.09.2014**
(21) Application number: 11002918.8
(22) Date of filing: 07.04.2011
(51) Int. Cl.: A61K 38/34, A61P 13/12, A61P 29/00

(54) **Melanocyte-stimulation hormone for suppressing inflammation reactions in hemodialysis**
Melanozytenstimulierendes Hormon zur Unterdrückung von Entzündungsreaktionen bei Hämodialyse
Hormone de stimulation de mélanocyte pour supprimer les réactions d'inflammation dans l'hémodialyse

(43) Date of publication of application: 10.10.2012
(73) Proprietor: Fresenius Medical Care Deutschland GmbH, 61352 Bad Homburg (DE)
(72) Inventor: Paßlick-Deetjen, Jutta, 35392 Giessen (DE); Fislage, Rainer, 66606 St. Wendel (DE); Kuhn, Anja, 80995 München (DE); Steppan, Sonja, 63263 Neu-Isenburg (DE)
(74) Representative: Weiß, Stefan

(56) References cited:
- WO-A1-03/094990
- WO-A1-2010/129248
- WO-A2-2004/104019
- US-A1- 2010 143 438
- KELLY ET AL: "Immobilized alpha-melanocyte stimulating hormone 10-13 (GKPV) inhibits tumor necrosis factor-alpha stimulated NF-kappaB activity", PEPTIDES, ELSEVIER, AMSTERDAM, vol. 27, no. 2, 1 February 2006 (2006-02-01), pages 431-437, XP005269001, ISSN: 0196-9781, DOI: DOI:10.1016/J.PEPTIDES.2005.03.062
- GATTI S ET AL: "Inhibitory Effects of the Peptide (CKPV)2 on Endotoxin-Induced Host Reactions", JOURNAL OF SURGICAL RESEARCH, ACADEMIC PRESS INC., SAN DIEGO, CA, US, vol. 131, no. 2, 1 April 2006 (2006-04-01) , pages 209-214, XP024952797, ISSN: 0022-4804, DOI: DOI:10.1016/J.JSS.2005.08.009 [retrieved on 2006-04-01]

## Description

Disclosed is a construct S-L-aMSH comprising a surface S of a solid support, a linker L and melanocyte-stimulation hormone aMSH, wherein aMSH is immobilized on the surface S through the linker L and wherein the linker L comprises a poly(alkyleneoxide) moiety. The invention further relates to a conjugate L-aMSH comprising such a linker L and aMSH. The construct S-L-aMSH is useful in dialysis treatment, particularly for the prevention and/or suppression of inflammatory responses and processes.

Dialysis is a method of cleansing an individual's blood when the kidneys fail to function properly. Dialysis eliminates the body's extra salt, wastes and water, and assists to control blood pressure.

Two types of dialysis are usually distinguished: hemodialysis and peritoneal dialysis. In hemodialysis, a patient is connected via tubes to an artificial kidney. Blood is pumped out of the body to the artificial kidney which filters the blood, and then the blood is returned to the body. In peritoneal dialysis, the inside lining of the patient's peritoneal cavity is used as a natural filter. Wastes are removed by means of a fluid, the dialysate, which is washed in and out of the peritoneal cavity in cycles.

In hemodialysis, an artificial kidney (hemodialyzer) is used to remove waste, extra chemicals and excess fluid from the blood. To transfer a dialysis patient's blood into the artificial kidney, vascular access is required. A common access is made by joining an artery to a vein under the skin and thus creating a fistula. The fistula is then penetrated, allowing access to blood. Occasionally, an access is made by means of a catheter, which is inserted into a large vein in the neck. This type of access may be temporary, but in many instances may be used for long-term treatment. As with any vascular access or catheter treatment, the catheter creates a direct communication between a patient's aseptic internal environment and the outside world. Common complications of this communication are infections, inflammations and thrombotic or infiltrative blockage of the access.

Inflammation represents a major medical complication associated with dialysis. In fact, although the benefits of hemodialysis and peritoneal dialysis are numerous, many of the complications associated with each may be life threatening. Each technique has its own or similar complications including, but not limited to, ultrafiltration failure, compliance, obesity, clearance, and poor fluid compliance.

Inflammation is a major factor for high mortality in patients with end stage renal disease (ESRD). It is considered that inflammation plays a primary role in arterial damage in dialysis patients. Although the precise mechanisms leading to this inflammatory state in ESRD are unclear, low-grade infection, repeated exposure to dialysis filters and auto-oxidation products are discussed as likely inciting factors in these patients (cf. C. Zocalli et al., Blood purification, 2003, 21, 29-36; and P. Stenvinkel et al., Semin. Dial., 2002, 15, 329-37).

There is a demand for methods that cause less inflammation in hemodialysis patients.

aMSH (melanocyte-stimulation hormone) derives from a precursor hormone, namely propiomelanocortin (POMC). The post-translational processing of POMC yields in peptide hormones such as ACTH, aMSH, gMSH and b-endorphin.

### aMSH has multiple effects on the host.

The stimulatory effect of aMSH in pigment cells has been known for over 50 years. More recent findings indicate that aMSH controls food-intake and exocrine secretions, modulates fever (cf. D. B. Richards et al., Peptides 1984, 5(4), 815-7) and inflammatory reactions and has an antimicrobial effect (cf. e.g. A. Catania et al., J Leukoc Biol. 2000, 67(2), 233-9). aMSH induces Fos expression in supraoptic oxytocin neurons (N. Sabatier et al., J. Neuroscience, 2003, 23(32), 10351-8).

[Nle4-d-Phe7] aMSH (NDP-MSH) has been described as a protease-stable aMSH analogue (cf. T. K. Sawyer et al., Proc. Natl. Acad. Sci. USA, 1980, 77(10), 5754-58).

US 2005/0130901 and US 2006/0122121 relate to peptides with antimicrobial activity. The peptides are octomeric peptides modified from aMSH.

WO 2004/004551 discloses a composition and method for controlling host response to organ and/or tissue transplantation and grafting. aMSH protects organ and tissue after transplantation by controlling factors within the donor, host and of the organ or tissue to be transplanted.

It is known that aMSH concentrations are elevated in patients on chronic hemodialysis. Concentrations of up to 30 ng/l are observed whereas healthy patients show concentrations of up to 25 ng/l only. The concentration of aMSH is elevated particularly when simultaneously an elevated concentration of endotoxin can be observed. Possibly, aMSH release is induced by cytokines in order to limit their effect (cf. L. Airaghi et al., Nephrol Dial Transplant, 2000, 15, 1212-6).

WO 03/094990 relates to oligo- and polysaccharides containing the sugar structural element N-acylglucosamine or N-acylgalactosamine, in addition to the use thereof for producing haemocompatible surfaces and methods for coating surfaces with said oligo- and polysaccharides, which constitute the common biosynthetic precursor substances of heparin, heparan sulfates and chitosan.

WO 2004/104019 discloses the use of αMSH for use in dialysis and in the treatment of inflammation.

Kelly *et al.* discloses an S-L-alphaMSH construct for use in treating inflammation (Kelly et al., "Immobilized alpha-melanocyte stimulating hormone 10-13 (GKPV) inhibits tumor necrosis factor- alpha stimulated NF- kappaB activity", PEPTIDES, 27 (2006) 431-437).

Gatti *et al.* discloses the use of alpha-MSH as well as of KPV for use in dialysis and in the treatment of inflammation. (Gatti et al., "Inhibitory Effects of the Peptide (CKPV)2 on Endotoxin-Induced Host Reactions", JOURNAL OF SURGICAL RESEARCH, 131 (2006) 209-214)

US 2010/143438 discloses an S-L-alphaMSH construct for use in treating inflammation.

WO 03/094990 relates to the coating of hemocompatible surfaces including dialysis devices.

WO 2010/129248 discloses an S-L-alphaMSH construct for use in treating inflammation.

These compositions, however, are not satisfactory in every respect and there is a demand for further improvements of hemodialysis.

It is an object of the invention to provide improvements for hemodialysis and other methods where body fluids are guided through extracorporeal circuits, particularly with respect to the occurrence of inflammation.

This object is achieved by the subject-matter of the patent claims.

It has been surprisingly found that surfaces that are modified with aMSH are particularly useful in hemodialysis with respect to the prevention and/or suppression of inflammation.

For the purpose of this invention aMSH may also be replaced by fractions of aMSH. Fractions may be peptides comprising an amino acid sequence of at least 3 amino acids which is also comprised in aMSH. A preferred peptide of this kind has or comprises the amino acid sequence KPV.

A first aspect of the invention relates to a construct S-L-aMSH comprising a surface S of a solid support, a linker L and melanocyte-stimulation hormone aMSH, wherein aMSH is immobilized on the surface S through the linker L for the use in dialysis treatment, preferably hemodialysis or peritoneal dialysis.

The construct according to the invention comprises a surface S of a solid support, a linker L and αMSH. Preferably, the construct S-L-αMSH consists of surface S of a solid support, linker L and αMSH, i.e., does not include further constituents.

The surface S of the solid support is not particularly limited, as long as it is capable for immobilization of αMSH through the linker L.

Preferably, the surface S is adapted for being contacted with a body fluid, such as blood, serum and plasma.

In a particularly preferred embodiment, the surface S is hemocompatible.

For the purpose of the specification hemocompatibility is preferably defined according to ISO 10993-4 relating to tests concerning the effects of blood contacting the product or compounds on blood or blood components, directly or indirectly during routine use.

Hemocompatibility is preferably achieved by providing the solid support with an external coating, e.g., a hemocompatible layer. The hemocompatible layer can be added directly onto the surface of a preferably non hemocompatible solid support or deposited onto other biostable and/or biodegradable layers. Additional biostable and/or biodegradable and/or hemocompatible layers can be present on top of the hemocompatible layer.

Hemocompatible materials are known to the person skilled in the art. Preferably, the surface S of the solid support is rendered hemocompatible *inter alia* by the presence of αMSH that is immobilized on the surface S through linker L.

It is preferred that at least one biostable layer is present under the hemocompatible layer. In addition the hemocompatible layer can be coated totally and/or partially with at least one more, above lying biostable and/or biodegradable layer.

Examples of biodegradable substances for the biodegradable layer(s) include polyvalerolactones, polylactonic acid, poly-[epsilon]-decalactones, polyglycolic acid, polylactides, polyglycolides, copolymers of the polylactides and polyglycolides, poly-[epsilon]-caprolactone, polyhydroxybutanoic acid, polyhydroxybutyrates, polyhydroxyvalerates, polyhydroxybutyrate-co-valerates, poly(1,4-dioxane-2,3-diones), poly(1,3-dioxane-2-one), poly-para-dioxanones, polyanhydrides such as polymaleic anhydrides, polyhydroxymethacrylates, fibrin, polycyanoacrylates, polycaprolactonedimethylacrylates, poly-b-maleic acid, polycaprolactonebutyl-acrylates, multiblock polymers such as e.g. from oligocaprolactonedioles and oligodioxanonedioles, polyether ester multiblock polymers such as e.g. PEG and poly(butyleneterephtalates), polypivotolactones, polyglycolic acid trimethyl-carbonates, polycaprolactone-glycolides, poly(g-ethylglutamate), poly(DTH-iminocarbonate), poly(DTE-co-DT-carbonate), poly(bisphenol-A-iminocarbonate), polyiminocarbonates, polyorthoesters, polyglycolic acid trimethyl-carbonates, polytrimethylcarbonates, poly(N-vinyl)-pyrrolidone, polyvinylalcoholes, polyesteramides, polyphosphoesters, glycolated polyesters, polyphosphazenes, poly[p-carboxyphenoxy)propane], polyhydroxypentanoic acid, polyanhydrides, poly-(ethyleneoxide-propyleneoxide), soft polyurethanes, polyurethanes with amino acid residues in the backbone, polyether esters such as polyethyleneoxide, polyalkene-oxalates, polyorthoesters as well as their copolymers, carrageenans, lipides, fibrinogen, starch, collagen, protein based polymers, polyamino acids, synthetic polyamino acids, zein, modified zein, polyhydroxyalkanoates, actinic acid, pectic acid, modified and non modified fibrin and casein, carboxymethylsulphate, albumin, moreover hyaluronic acid, chitosan and its derivatives, heparansulphates and its derivatives, heparins, chondroitinsulphate, dextran, b-cyclodextrins, copolymers with PEG and polypropyleneglycol, gummi arabicum, guar, gelatine, collagen, collagen-N-hydroxysuccinimide, lipids, phospholipids, modifications and copolymers and/or mixtures of the afore mentioned substances.

Examples of biostable substances for the biostable layer(s) include polyacrylic acid and polyacrylates as polymethylmethacrylate, polybutylmethacrylate, polyacrylamide, polyacrylonitriles, polyamides, polyetheramides, polyethylenamine, polyimides, polycarbonates, polycarbourethanes, polyvinylketones, polyvinylhalogenides, polyvinylidenhalogenides, polyvinylethers, polyisobutylenes, polyvinylaromates, polyvinylesters, polyvinylpyrollidones, polyoxymethylenes, polytetramethyleneoxide, polyethylene, polypropylene, polytetrafluoroethylene, polyurethanes, polyetherurethanes, silicone-polyetherurethanes, silicone-polyurethanes, silicone-polycarbonate-urethanes, polyolefine elastomeres, polyisobutylenes, EPDM gums, fluorosilicones, carboxymethylchitosanes, polyaryletheretherketones, polyetheretherketones, polyethylenterephthalate, polyvalerates, carboxymethylcellulose, cellulose, rayon, rayontriacetates, cellulosenitrates, celluloseacetates, hydroxyethylcellulose, cellulosebutyrates, celluloseacetatebutyrates, ethylvinylacetate copolymers, polysulphones, epoxy resins, ABS resins, EPDM gums, silicones as polysiloxanes, polydimethylsiloxanes, polyvinylhalogenes and copolymers, celluloseethers, cellulosetriacetates, chitosanes and copolymers and/or mixtures of these substances.

In a preferred embodiment, the surface S contains at least an area with a surface concentration of αMSH of at least 1.0 nmol/cm², more preferably at least 2.0 nmol/cm², still more preferably at least 5 nmol/cm², yet more preferably at least 10 nmol/cm², most preferably at least 25 nmol/cm² and in particular at least 50 nmol/cm². Preferably, said area is at least 1.0 cm² large.

The surface S is the surface of a solid support. The solid support is not particularly limited. Preferably, the solid support is a medical device, e.g. component of an extracorporeal circuit, preferably of a system or component thereof adapted for hemodialysis, hemofiltration, plasmapheresis, apheresis, extracorporeal membrane oxygenation or assisted blood circulation.

In a preferred embodiment the surface S of the solid support is suitable for the short- or the longterm contact with blood or blood products. Examples of solid supports include medical devices such as prostheses, organs, vessels, aortas, heart valves, tubes, organ spareparts, implants, fibers, hollow fibers, stents, hollow needles, syringes, membranes, tinned goods, blood containers, titrimetric plates, pacemakers, adsorbing media, chromatography media, chromatography columns, dialyzers, connexion parts, sensors, valves, centrifugal chambers, recuperators, endoscopes, filters, and pump chambers. Dialyzers are particularly preferred, especially their components, particularly dialysis membranes.

The solid support may be composed of inorganic and/or organic material, metal or polymers. Suitable polymers may be thermoplastic and are known to the skilled artisan.

The solid support and the surface S, respectively, can assume any shape, e.g. plain, convex, concave and the like. When the solid support is a dialysis membrane, it may be, e.g., fibrous, tissue-like, a hollow fiber, or a flat membrane.

Linkers L that are suitable to immobilize αMSH on a suitable surface S are known to the skilled person. In this regard it can be referred e.g. to G.T. Hermanson, Bioconjugate Techniques, Second Edition, Academic Press 2008; F. Zaragoza Dörwald, Organic Synthesis on Solid Phase: Supports, Linkers, Reactions, Wiley-VCH; 2 edition 2002; J. M. Guisan, Immobilization Of Enzymes And Cells (Methods in Biotechnology), Humana Press; 2 edition 2006; J.A. Camarero, Biopolymers. 2008, 90(3), 450-8.

The chemical nature of the linker L is not particularly limited. Typical linkers L comprise at least one distal end that is faced to αMSH and at least one proximal end that is faced to the surface S.

The linker L may have any end-to-end distance from the distal end to the proximal end that is suitable in order to immobilize αMSH on the surface S of the solid support. Typical (average) lengths are in the range of from a few nanometers to several nanometers or more.

Preferably, the linker L is an organic molecule. It may contain an uninterrupted carbon-carbon chain between the distal end and the proximal end. Preferably, however, in between at least some of the carbon atoms of the chain are heteroatoms which are preferably independently selected from Si, N, S and O.

Preferably, linker L is substantially linear. Preferably, linker L comprises a moiety that is composed of repetition units, like in a polymer or oligomer. For example, when linker L comprises a polyethylene glycol moiety, the repetition units are -(O-CH₂CH₂)ₓ-.

The linker L may be derived from biomolecules, such as peptides, or synthetic, such as polyalkylene glycols, e.g., polyethylene glycols or polypropylene glycols.

The linker L may be composed of several segments that differ from one another in chemical nature but are covalently linked with one another. For example, a first segment of linker L may be peptidic and a second segment of linker L that is covalently bound to said first segment may be a polyalkylene glycol or a polyalkylene oxide.

The linker L comprises a poly(alkylene glycol) a poly(alkyleneoxide) segment or moiety.

In a preferred embodiment the linker L does not include any α-amino acid residue, i.e., the linker is non-peptidic.

In a preferred embodiment, the overall molecular weight of the linker L is within the range of from 500 g/mol to 2,000,000 g/mol, more preferably 750 g/mol to 1,000,000 g/mol, still more preferably 1000 g/mol to 500,000 g/mol, yet more preferably 1250 g/mol to 100,000 g/mol, most preferably 1500 g/mol to 50,000 g/mol and in particular 2000 g/mol to 10,000 g/mol.

In a preferred embodiment, the surface S comprises free carboxyl groups (-COOH) which are activated by the use of EDC or other activation compounds. Subsequently, this activated surface S is preferably brought in contact with an Amino-PEG -modified αMSH to yield the corresponding covalent bond.

The linker L is preferably covalently bound to the surface S, e.g. via an ester bond, ether bond, amide bond, thioether bond, and the like. Chemoselective immobilizations may be achieved, e.g., by amino group to aldehyde linkage, aminooxyacetyl group to glyoxylyl linkage, cysteine residue to thioester linkage, cyclopentadiene residue to benzoquinone linkage, and the like.

Solid supports bearing at their surfaces S reactive functional groups that are capable of reacting with a compatible functional group at the proximal end of liker L are commercially available. For example, the solid supports may bear at their surfaces S reactive functional groups selected from the group consisting of amines, thiols, aldehydes, carboxylic acids, aminooxyacetyl groups, glyoxylyl groups, thioester groups, cyclopentadiene groups, benzoquinones, trialkoxysilanes, and the like.

Methods for introducing said reactive functional groups to the surfaces S of solid supports are also known to the person skilled in the art.

Preferably, the linker L and the surface S of the solid support are linked with one another only by covalent bonds so that between αMSH and surface S there is a continuous chain of covalent bonds, which chain may be linear or branched.

It is also possible that the linker L is non-covalently bound to the surface S, e.g. by biotin-(strept)avidin interaction and the like. Under these circumstances the solid support bears at its surface S, e.g., biotin molecules that are attached to the surface S by conventional methods. The proximal end of the linker L in turn bears a compatible (strept)avidine group so that a molecular complex can be formed thereby immobilizing the linker L at the surface S. A skilled person recognizes that the location of the biotine and the (strept)avidine at surface S and linker L can be exchanged. Biotin-labelling can be easily achieved, e.g., by biotin-pegylation.

The linker L is preferably covalently bound to αMSH, e.g. via an ester bond, ether bond, amide bond, thioether bond, and the like. Such a linkage may be achieved, e.g., by amino group to aldehyde linkage, cysteine residue to thioester linkage, amide linkage and the like.

Linkers L bearing at their distal ends reactive functional groups that are capable of reacting with a compatible functional group at αMSH are commercially available. For example, the linkers L may bear at their distal ends reactive functional groups selected from the group consisting of amines, thiols, alcohols, aldehydes, carboxylic acids, aminooxyacetyl groups, glyoxylyl groups, thioester groups and the like.

It is also possible that the linker L is non-covalently bound to αMSH, e.g. by biotin-(strept)avidin interaction and the like. Under these circumstances the linker L bears at its distal end, e.g., biotin molecules that are attached to the linker L by conventional methods. The αMSH in turn bears a compatible (strept)avidine group so that a molecular complex can be formed thereby attaching αMSH to the distal end of the linker L. A skilled person recognizes that the location of the biotine and the (strept)avidine at αMSH and linker L can be exchanged.

Preferably, every linker L bears one molecule of αMSH. It is also possible however, that more than one molecule of αMSH is linked to a single linker L. This can be achieved, for example, by means of branched linkers L that bear one proximal end attached to the surface S of the solid support and more than one distal end, e.g. 2, 3 or 4 distal ends, linked to one molecule of αMSH each.

In a preferred embodiment, the construct S-L-αMSH according to the invention comprises a sub-structure -L-αMSH according to general formula (I)

-K-[CH2-CH2-O]n-[CH2]m-Q-αMSH (I)

wherein
Q is a bond or a functional group selected from -O-, -NH-, -C(=O)-, -C(=S)-, -C(=NH)-, -NH-C(=O)-, -NH-C(=S)- and -NH-C(=NH)-;
K is a bond or a functional group selected from -NH-, -O-, -S-, -C(=O)-, - C(=S)-,
-C(=NH)-, -NH-C(=O)-, -NH-C(=S)-, -NH-C(=NH)-, -NH-C(=O)-O-, -NH-C(=O)-NH-,
-O-C(=O)-O- or -Si(R')₂-, wherein R' is -C₁₋₆-alkyl or -O-C₁₋₆-alkyl;
n is an integer of from 0 to 250, preferably 1 to 25, more preferably 2 to 10; and
m is an integer of from 0 to 12, preferably 1, 2 or 3.

A further aspect described herein relates to a conjugate L-αMSH comprising a linker L as defined above covalently bound to αMSH.

The linker L comprises a polyalkylene glycol moiety, particularly preferred a polyethylene glycol moiety (peg) such that the conjugate L-αMSH preferably can be regarded as α-MSH that is pegylated by the polyethylene glycol moiety of linker L.

The conjugate L-aMSH according to the invention can have a structure according to general formula (II)

H-K-[CH₂-CH₂-O]ₙ-[CH₂]ₘ-Q-a-MSH (II)

wherein
Q is a bond or a functional group selected from -O-, -NH-, -C(=O)-, -C(=S)-, -C(=NH)-, -NH-C(=O)-, -NH-C(=S)- and -NH-C(=NH)-;
K is a bond or a functional group selected from -C₁₋₈-alkylene-, -NH-, -O-, - S-,
-C(=O)-, or -C(=O)O-;
n is an integer of from 0 to 250; and
m is an integer of from 0 to 12;

For the purpose of the specification, bivalent functional groups that have two possibilities of being incorporated in a general formula in two different directions, e.g. -NH-C(=O)-, can be incorporated in both directions alternatively.

It is preferred that Q is -C(=O)-, m is 2, n is 3 and K is -NH-, so that the conjugate L-P has the following structure (III)

H₂N-CH₂-CH₂-O-CH₂-CH₂-O-CH₂-CH₂-O-CH₂-CH₂-C(=O)-aMSH (III).

The conjugate L-αMSH according to the invention may be employed as an intermediate in the preparation of the construct S-L-αMSH according to the invention by attaching the conjugate L-αMSH to the surface S of the solid support be appropriate methods.

Also described is a process for the manufacture of the construct S-L-αMSH comprising the step of linking αMSH as defined above to a surface S of a solid support as defined above through a linker L as defined above.

In a preferred embodiment, the process comprises the step of linking a conjugate L-αMSH as defined above to a surface S of a solid support as defined above.

In the process, the proximal end of linker L bears a functional group that is adapted for covalent or non-covalent immobilization of linker L at surface S. For that purpose, surface S typically bears a functional group that is compatible with the functional group at the proximal end of linker L so that a stable linkage can be achieved, optionally after chemical activation of one or both of the functional groups.

Suitable functional groups and activation methods are known to the skilled person and have been described above in connection with linker L and surface S, respectively.

The construct S-L-αMSH is useful for dialysis such as hemodialysis and peritoneal dialysis, hemofiltration, plasmapheresis, apheresis, extracorporeal membrane oxygenation or assisted blood circulation.

Preferably, it is used to prevent and/or suppress inflammation, preferably in patients in the course of dialysis, hemofiltration, plasmapheresis, apheresis, extracorporeal membrane oxygenation or assisted blood circulation, preferably in patients suffering from ESRD.

The following examples further illustrate the invention but are not to be construed as limiting its scope.

### Example 1:

### Ex vivo stimulation of human peripheral blood mononuclear cells (PBMCs)

PBMCs from healthy male volunteers (n=5) were isolated and stimulated as indicated below to mimic an uremic and inflammatory setting comparable to patients (LPS pre-incubation for 3 hours)

### Material and Methods

For the stimulation of PBMCs the following concentrations were chosen:
- LPS 1 ng/ml alone
- αMSH
   ∘ 1µM; 10µM; 100µM
- Peg-KPV (pegylated tripeptide)
   ∘ 1µM; 10µM; 100µM; 1000µM

The stimulation procedure was as described below:

| Pre-incubation LPS | Co-stimulation LPS+Peptides |
|---|---|
| yes 3h | 24h |
| no | 24h |

All cells were incubated for 24h and centrifuged at 1700 rpm and supernatants were used for TNF-α ELISA measurement according to manufacturer's protocols.

### Results

The results of the pre-stimulation are summarized in the following table and are additionally depicted in Figures 1A and 1B:

| | TNF-a | LPS 3 h pre-incubation | | | | | | | |
|---|---|---|---|---|---|---|---|---|---|
| | | Donor 2 | Donor 3 | Donor 4 | Donor 5 | Donor 6 | MV+SD | SD | % |
| c | c | 4808 | 4690 | 4400 | 3157 | 4897 | 4390.4 | 714.521028 | 100.00 |
| αMSH Sigma | 1 | 5005 | 4345 | 3968 | 2070 | 4286 | 3934.8 | 1108.54801 | 89.62 |
| | 10 | 4193 | 3883 | 3674 | 1799 | 4356 | 3581 | 1030.84262 | 81.56 |
| | 100 | 3009 | 2886 | 2073 | 942 | 2069 | 2395.8 | 906.537754 | 54.57 |
| KPV [µM] | 1 | 4025 | 4554 | 3541 | 2301 | 4299 | 3744 | 889.739288 | 85.28 |
| | 10 | 4126 | 4387 | 3666 | 2209 | 3949 | 3667.4 | 856.500029 | 83.53 |
| | 100 | 3542 | 5022 | 2886 | 1931 | 3731 | 3222.4 | 833.825102 | 73.40 |
| | 1000 | not | 1816 | 1494 | 1054 | 2482 | 1711.5 | 601.166366 | 38.98 |

| | | | | | | | | | |
|---|---|---|---|---|---|---|---|---|---|
| | | done | | | | | | | |
| Peg-KPV | 1 | 4082 | 4092 | 6032 | 3241 | 5201 | 4529.5 | 1090.9071 | 103.17 |
| | 10 | 4129 | 4265 | 5243 | 2875 | 4599 | 4222.2 | 867.392184 | 96.17 |
| | 100 | 4185 | 3610 | 4993 | 2435 | 3859 | 3816.4 | 931.790642 | 86.93 |
| | 1000 | not done | 1647 | 2224 | 2467 | 2467 | 1869 | 596.454525 | 42.57 |

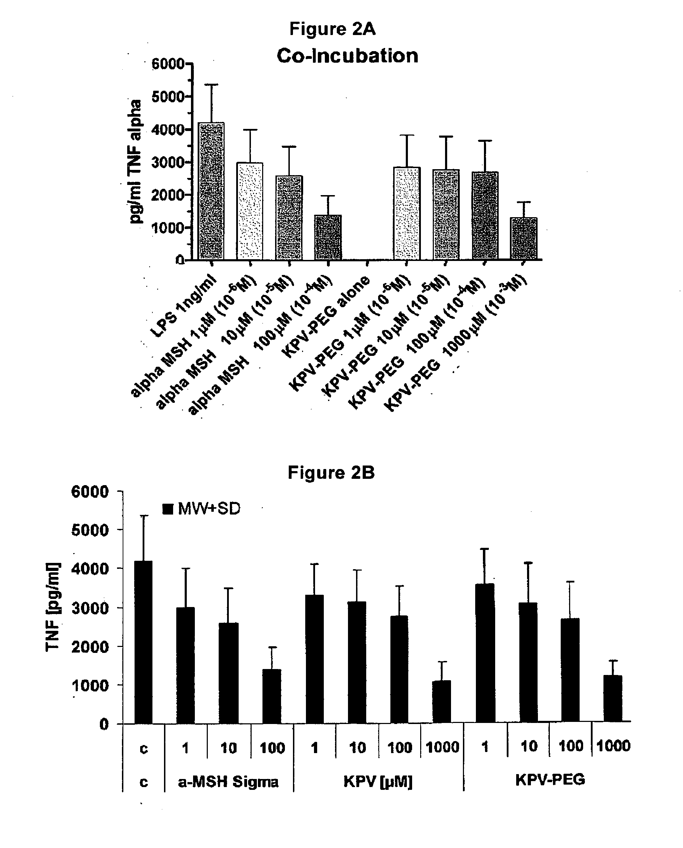

The results of the co-incubation are summarized in the following table and are additionally depicted in Figures 2A and 2B:

| | TNF-a | LPS + peptide simultaneously | | | | | | | |
|---|---|---|---|---|---|---|---|---|---|
| | | Donor 2 | Donor 3 | Donor 4 | Donor 5 | Donor 6 | MV+SD | SD | % |
| C | C | 4463 | 5268 | 4749 | 2207 | 4316 | 4200.6 | 1172.25181 | 100.00 |
| αMSH Sigma | 1 | 4098 | 3273 | 3116 | 1319 | 3100 | 2981.2 | 1015.82267 | 70.97 |
| | 10 | 3734 | 2776 | 2568 | 1237 | 2638 | 2590.6 | 891.168783 | 61.67 |
| | 100 | 1207 | 2337 | 1248 | 713 | 1386 | 1380.2 | 593.368941 | 32.86 |
| KPV [µM] | 1 | 3426 | 3955 | 3702 | 1893 | 3541 | 3303.4 | 813.046309 | 78.64 |
| | 10 | 3154 | 3907 | 3369 | 1706 | 3470 | 3121.2 | 837.319154 | 74.30 |
| | 100 | 2562 | 3505 | 3077 | 1525 | 3142 | 2762.2 | 769.064822 | 65.76 |
| | 1000 | not done | 931 | 934 | 594 | 1801 | 1065 | 515.963177 | 25.35 |
| Peg-KPV | 1 | 3893 | 3697 | 3918 | 2021 | 4340 | 3573.8 | 899.137198 | 85.08 |
| | 10 | 3918 | 3274 | 2846 | 1317.5 | 3909 | 3052.9 | 1070.58526 | 72.68 |
| | 100 | 3617 | 3067 | 2305 | 1110.5 | 3088 | 2637.5 | 973.283874 | 62.79 |
| | 1000 | not done | 971 | 1428 | 694.5 | 1586 | 1169.875 | 410.414907 | 27.85 |

The data indicate an inhibitory effect for KPV and peg-KPV (provided by Invitrogen Life Technolgies) as well as αMSH for LPS-induced TNF-α release ("inflammation") when the inflammatory process has already started (pre-stimulation) or if the peptides and inflammatory stimulus is given simultaneously (co-incubation). The pre-incubation of LPS was used as a model to mimic an uremic situation in patients, in which inflammatory processes are ongoing already.

### Example 2:

Whole blood studies with blood from uremic patients including heat sterilization experiments

A first goal of this experiment was to test whether the three amino acid antiinflammatory peptide KPV (unpegylated and pegylated version "monopeptide") will retain its activity under conditions of steam sterilization at 125°C for 15 min used in the production process of dialysis modules. A second goal was to study the anti-inflammatory effect of αMSH and the peptides in uremic patients, which represent the real clinical situation.

Since peptides tend to lose activity when heated the experiment was designed to mimic the dialysis production process with the known parameters.

To perform this experiment whole blood samples were taken from voluntary uremic patients (n=10) and subsequently stimulated ex vivo with lipopolysaccharide (LPS) with or without αMSH, KPV or pegylated KPV (non heated and heated) and TNF-α and IL-6 release was determined by ELISA.

### Material and Methods

25 ml blood from 10 uremic patients (dialysis patients treated at the hospital in Konstanz, Germany) was drawn and blood values were analyzed immediately. Blood was 1:10 diluted with PBS buffer and each stimulation condition was performed in triplicates and incubated at 200 µl in 96 well plates.

The following conditions were chosen:
- Unstimulated blood
- Blood stimulated with LPS 1 ng/ml alone (maximal stimulation of proinflammatory cytokine TNF-α.
- Blood stimulated with LPS 1 ng/ml and 4 different concentrations of α MSH
   o 0.1µM;1µM;10µM;100µM
- Blood stimulated with LPS 1 ng/ml and 5 different concentrations of
   o KPV (0.1 µM; 1 µM; 10µM; 100µM; 1000µM)
   o KPV-peg (0.1µM; 1µm; 10µM; 100µM; 1000µM)
- Blood stimulated with LPS 1 ng/ml and 5 different concentrations of
   o KPV heated(0.1µM; 1µM; 10µM; 100µM; 1000µM)
   o KPV-peg heated (0.1µM; 1µM; 10µM; 100µM; 1000µM)

Blood was incubated for 24 h, centrifuged at 1700 rpm and serum supernatants were used for TNF-α and IL-6 ELISA measurement according to manufacturer's protocols.

### Results

TNF-α and IL-6 ELISA showed that the heat treatment did not influence the activity of the peptide. The average inhibition for the tested concentrations of KPV for TNF-α was around 30% and for and IL-6 around 20%.

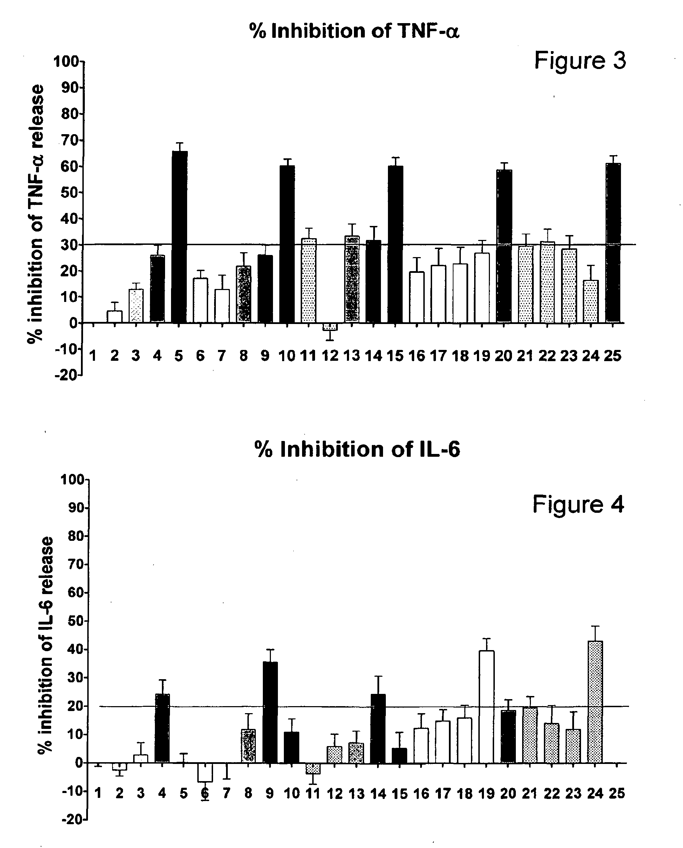

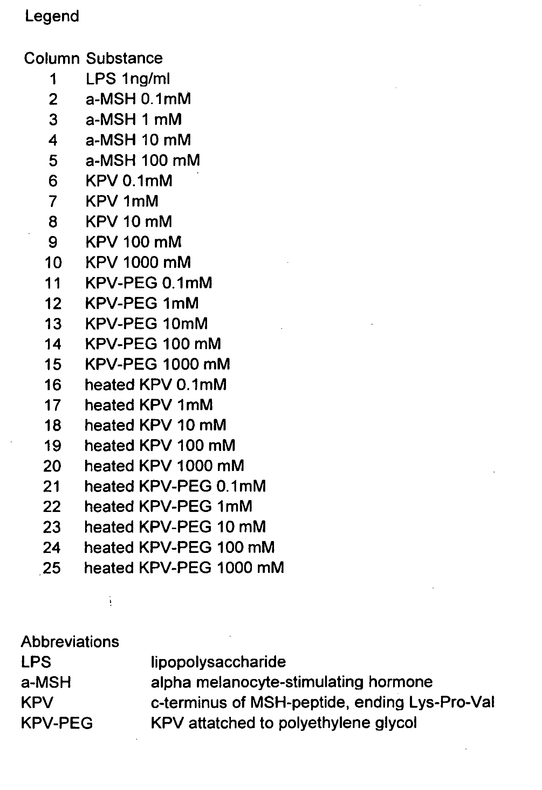

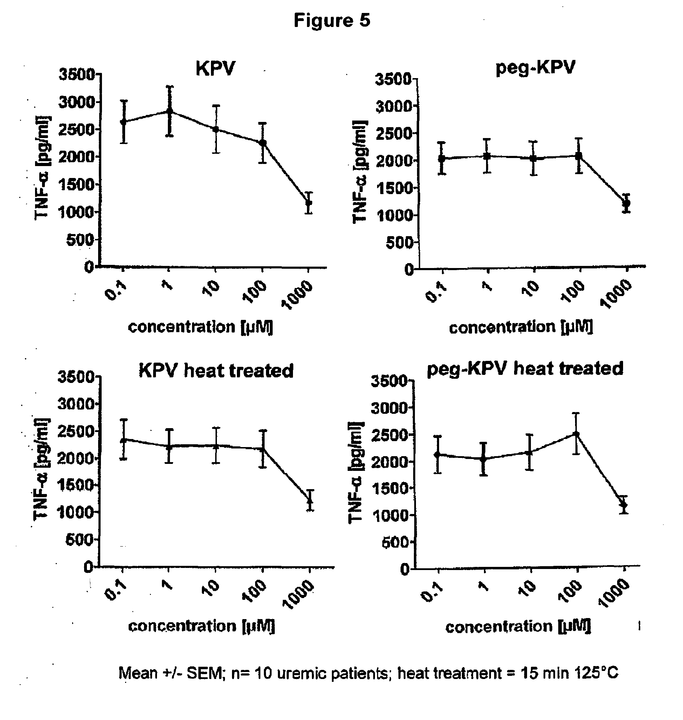

The results are further depicted in Figures 3, 4 and 5.

## Claims

1. A construct S-L-αMSH comprising a surface S of a solid support, a linker L and melanocyte-stimulation hormone αMSH, wherein αMSH is immobilized on the surface S through the linker L and wherein the linker L comprises a poly(alkyleneoxide) moiety for the use in dialysis treatment, preferably hemodialysis treatment.

2. The construct for use according to claim 1, wherein the linker L does not include any a amino acid residue.

3. The construct for use according to any of the preceding claims, wherein the surface S is adapted for being contacted with a body fluid.

4. The construct for use according to any of the preceding claims, wherein the solid support is a component of an extracorporeal circuit.

## Patentansprüche

1. Konstrukt S-L-αMSH, umfassend eine Oberfläche S eines festen Trägers, einen Linker L und ein Melanozyten stimulierendes Hormon αMSH, wobei αMSH auf der Oberfläche S durch den Linker L immobilisiert ist und wobei der Linker L eine Poly(alkylenoxid)-Einheit zur Verwendung bei der Dialysebehandlung, vorzugsweise der Hämodialysebehandlung umfasst.

2. Konstrukt zur Verwendung nach Anspruch 1, wobei der Linker L keinen α-Aminosäurerest aufweist.

3. Konstrukt zur Verwendung nach einem der vorhergehenden Ansprüche, wobei die Oberfläche S geeignet ist, mit einer Körperflüssigkeit in Kontakt gebracht zu werden.

4. Konstrukt zur Verwendung nach einem der vorhergehenden Ansprüche, wobei der feste Träger ein Bestandteil eines extrakorporalen Kreislaufs ist.

## Revendications

1. Construction S-L-αHSM comprenant une surface S d'un support solide, une séquence de liaison L et une hormone stimulatrice des mélanocytes αHSM, dans laquelle αHSM est immobilisée sur la surface S via la séquence de liaison L et dans laquelle la séquence de liaison L comprend une fraction poly(alkylènoxyde) à utiliser dans un traitement par dialyse, de préférence un traitement par hémodialyse.

2. Construction à utiliser selon la revendication 1, dans laquelle la séquence de liaison L n'inclut pas de résidu d'acides aminés quelconques.

3. Construction à utiliser selon l'une quelconque des revendications précédentes, dans laquelle la surface S est adaptée pour être en contact avec un fluide corporel.

4. Construction à utiliser selon l'une quelconque des revendications précédentes, dans laquelle le support solide est un composant d'un circuit extracorporel.
